# EUROPÄISCHE PATENTSCHRIFT

(11) **EP 1 430 098 B1**
(45) Veröffentlichungstag und Bekanntmachung des Hinweises auf die Patenterteilung: **12.10.2011**
(21) Anmeldenummer: 02797599.4
(22) Anmeldetag: 21.08.2002
(51) Int. Cl.: C08L 33/02, C08L 39/06, A61L 15/58, C08J 3/02, A61K 47/10

(54) **GELMATRIX AUS POLYACRYLSÄURE UND POLYVINYLPYRROLIDON**
GEL MATRIX CONSISTING OF POLYACRYLIC ACID AND POLYVINYL PYRROLIDONE
MATRICE DE GEL COMPRENANT DE L'ACIDE POLYACRYLIQUE ET DE LA POLYVINYLPYRROLIDONE

(30) Priorität: 01.09.2001 DE 10142918
(43) Veröffentlichungstag der Anmeldung: 23.06.2004
(73) Patentinhaber: Beiersdorf AG, 20245 Hamburg (DE)
(72) Erfinder: WÖLLER, Karl-Heinz, 20257 Hamburg (DE); NIERLE, Jens, 21147 Hamburg (DE)
(86) Internationale Anmeldenummer: PCT/EP2002/009311
(87) Internationale Veröffentlichungsnummer: WO 2003/020824

(56) Entgegenhaltungen:
- EP-A- 1 142 588
- WO-A-97/33645
- DE-A- 4 238 263
- CHEMICAL PATENTS INDEX, DOCUMENTATION ABSTRACTS JOURNAL Section Ch, Week 199837 Derwent Publications Ltd., London, GB; Class A14, AN 1998-434191 XP002224164 & JP 10 183084 A (TOMBOW PENCIL CO LTD), 7. Juli 1998 (1998-07-07)
- BEKTUROV E A ET AL: "SWELLING BEHAVIOUR OF A NON-IONIC POLY(N-VINYL-2-PYRROLIDONE) GEL IN A LINEAR POLY(ACRYLIC ACID) SOLUTION" MACROMOLECULAR CHEMISTRY AND PHYSICS, WILEY VCH, WEINHEIM, DE, Bd. 200, Nr. 2, Februar 1999 (1999-02), Seiten 431-435, XP000804483 ISSN: 1022-1352

## Beschreibung

Gegenstand der Erfindung ist eine selbstklebende Gelmatrix, insbesondere eine monolithische Gelmatrix, auf Polyacrylsäurebasis, die als Vemetzungsagenz Polyvinylpyrrolidon (PVP) enthält. Die Gelmatrix kann mit hydrophilen, bei geeignetem Lösungsvermittler auch hydrophoben, Wirkstoffen zur kosmetischen und/oder pharmazeutischen Behandlung der Haut oder systemischen Verabreichung von Arzneimitteln dotiert werden.

Die Herstellung von Gelmatrices aus Polyacrylaten ist seit vielen Jahren bekannt und wird z. B. in EP 0 507 160, JP 11-228340 und JP 04178323 beschrieben. Gelmatrices werden u.a. als Klebgrundlage und Wirkstoffreservoir in transdermalen Systemen eingesetzt. Eine in der Fachliteratur gut beschriebene Ausführungsform transdermaler Systeme stellen Matrixsysteme oder monolithische Systeme dar, in denen der Arzneistoff direkt in den druckempfindlichen Haftklebstoff eingearbeitet wird. Eine solche haftklebrige, wirkstoffhaltige Matrix ist im anwendungsfertigen Produkt auf der einen Seite mit einem für den Wirkstoff undurchlässigen Träger ausgestattet, auf der gegenüberliegenden Seite befindet sich eine mit einer Trennschicht ausgestatten Trägerfolie, die vor der Applikation auf die Haut entfernt wird (kleben&dichten, Nr.42, 1998, S. 26 bis 30).
Die beschriebenen Matrices haben jedoch in der Regel eine nur geringe Eigenklebkraft, so dass zur dauerhaften Fixierung auf der Haut eine zusätzliche klebende Applikationshilfe nötig ist. Oder die Systeme haben eine ausreichende Klebkraft, speziell auf feuchter Haut (Bukkalpflaster), lassen sich aufgrund ungenügender Kohäsivität bei Bedarf aber nicht vollständig wieder abziehen.

Polyacrylsäure muss zur Ausbildung eines Gels mit definierter Struktur vernetzt werden. Die Natur des Vernetzers trägt dabei entscheidend zur Struktur des resultierenden Gels bei. Die üblichen vernetzende Agentien können dabei Metallionen (z.B.: Al³⁺-Ionen), oder organische Verbindungen sein. Die Vernetzung mit Aluminiumsalzen läuft über die Koordination der Sauerstofffunktionen der Polyacrylsäure an die Al³⁺-Ionen. Es bildet sich ein sehr engmaschiges Gel mit hoher Viskosität aus, wobei die Viskosität des Gels nur über die Menge an Vernetzer gesteuert werden kann (handbook of pressure sensitive adhesive technology, Seite 458 ff, 1999).
In JP 11-228340 werden Gele auf Polyacrylsäurebasis offenbart, die als Vernetzer Al⁺³-Verbindungen nutzen. Der Einsatz der zwingend notwendigen Aluminiumverbindung als Vernetzungsagenz ist begrenzt, da ansonsten die physikalischen Eigenschaften des Geles verschlechtert werden. Bei zu hohem Anteil an Aluminiumvemetzer wird das Gel zu hart.

Aus der Literatur sind weitere Beispiele der Vernetzung mit multivalenten Metallionen bekannt, z.B. US 3900610 (Zinksalze), US 3770780 oder US 3790533 (Titanverbindungen). Die ionische Vernetzung mit Metallionen führt zu harten, viskosen und wenig klebrigen Polymergelen (handbook of pressure sensitive adhesive technology, Seite 458 ff, 1999).

Ein weiteres Problem bei der Vernetzung von Polyacrylsäure zu einem selbstklebenden Gel ist, dass ein einmal hergestelltes Gel mit definierten physikalischen Eigenschaften, Viskosität, Klebrigkeit etc. in einem späteren Herstellungsprozess die gleichen definierten Eigenschaften aufweisen muss. Diese Reproduzierbarkeit ist mit den derzeit bekannten Vernetzungstechnologien aufwendig oder gar nicht zu verwirklichen.

WO 97133645 offenbart selbstklebende Gelmatrices auf Polyacrylsäurebasis. Die Gele werden hergestellt indem Polycrylsäurepolymere über Amin-haltige Polymere mit Polyvinylpyrrolidon (PVP) vernetzt werden.

In EP 303445 wird ein Pflaster mit monolither Gelmatrix auf Basis wasserlöslicher Polymere offenbart. Als zwingend erforderliche Bestandteile sind Cleboprid oder ein pharmazeutisch akzeptables Salz davon als Wirkstoff, Wasser, wasseraufnehmende Agentien und wasserlösliche Polymere vorgesehen. Als wasserlösliche Polymere kann der Fachmann aus einer Reihe bekannter Polymere wie Polyvinylalkohol, Gelatine, Polyacrylsäure, Natriumpolyacrylate, Methylcellulose, Carboxymethylcellulose, Polyvinylpyrrolidon, Gummi und anderen vernetzbare Polymeren sowie Mischungen daraus auswählen.

Zum Unterschied zur erfindungsgemäßen Gelmatrix wird PVP als eine Möglichkeit des wasserlöslichen Polymers offenbart, jedoch nicht als Vernetzer für Polyacrylsäurebasierende selbstklebende Gele. Als Problem ist auch in EP 303445 die Viskositätsminderung und Klebkrafteinbuße der Polymere durch Änderung der Zusammensetzung, insbesondere des Vernetzungsagenz, dargestellt.

Aufgabe der Erfindung ist es, die aus dem Stand der Technik bekannten Nachteile zu vermeiden und eine selbstklebende Gelmatrix zur Verfügung zu stellen, deren Klebrigkeit, Kohäsivität und Viskosität individuell auf den jeweiligen Anwendungsbereich eingestellt werden kann. Insbesondere ist es die Aufgabe der Erfindung eine selbstklebenden Gelmatrix für transdermale Systeme bereit zu stellen, die für eine monolithische Pflasteranwendung nötige Klebkraft mit der entsprechenden Kohäsivität verbindet.

Gelöst wird diese Aufgabe durch eine Gelmatrix, wie sie im Anspruch 1 niedergelegt ist. Gegenstand der Unteransprüche sind dabei vorteilhafte Weiterbildungen der Gelmatrix.

Bei der erfindungsgemäßen Gelmatrix wird die Vernetzung der Polyacrylsäure mit Hilfe von Polyvinylpyrrolidon (PVP) durchgeführt.
Die Vernetzung läuft über die Bildung eines quartären Ammoniumsalzes des PVP. Diese Art der Vernetzung führt zu organischen Salzen, die im Gegensatz zu den bekannten Metallsalzen als Vemetzungsagentien über die Hydroxyfunktionen der Polyacrylsäuremoleküle gebunden werden. Wie bei den Metallsalzen ist die Reaktion reversibel und kann durch die Zugabe von Wasser oder Säuren umgekehrt werden. Überraschenderweise lässt sich die Viskosität des resultierenden Gels nicht nur über die Menge an Vernetzer steuern, sondern auch über das Molekulargewicht des PVP's. Dabei führen hohe Molekulargewichte zu Gelen mit niedriger Viskosität und niedrige Molekulargewichte zu Gelen mit hoher Viskosität und Klebkraft. Der Vorteil bei der erfindungsgemäßen Art der Vernetzung liegt somit darin, über die Parameter Anteil-PVP und Molekulargewicht-PVP zielgerichtet Gelmatrices herzustellen, deren Klebrigkeit, Kohäsivität und Viskosität individuell auf den jeweiligen Anwendungsbereich einstellbar sind.

Dieser Effekt des Einflusses des Molekulargewichtes des PVP auf die Viskosität und Klebkraft der Gelmatrix ist auf folgende Erkenntnis zurückführen: Bei langkettigem PVP ist die Zahl an Pyrrolidon-Untereinheiten pro Makromolekül deutlich höher als bei kurzkettigem PVP. Dadurch kommt es vermehrt zu Reaktionen der gleichen Reaktionspartner untereinander, da die Makromoleküle sich leicht zu Bündeln orientieren können. Diese Reaktionen führen nicht zur Bildung von Verknüpfungspunkten mit mehreren Polyacrylsäuremolekülen. Es werden daher nur wenige Querverbindungen zu anderen Polyacrylsäuremolekülen und damit nur wenige, große Maschen geknüpft. Dieser Umstand führt zu einem locker verknüpften Gel mit niedriger Viskosität. Im Gegensatz dazu werden bei kurzkettigem PVP aufgrund der höheren Beweglichkeit und der niedrigeren Tendenz zur Orientierung der Moleküle zu Strängen mehr Verknüpfungen zu verschiedenen Polyacrylsäuremolekülen gebildet, die zu einer engeren Maschenweite und einer geringeren Flexibilität und Viskosität des Gels führen.

Die Viskosität der Gele lässt sich darüber hinaus noch über andere Faktoren steuern. So spielt beispielsweise die Menge an PVP eine mitentscheidende Rolle für die Struktur des Gels. Wird ein Sättigungspunkt überschritten, so kommt es zu konkurrierenden Reaktionen der freien PVP Moleküle mit den bereits vernetzten. Diese Konkurrenzreaktionen führen dazu, dass Vernetzungspunkte zugunsten von nicht verknüpften Aggregaten aus Polyacrylsäure und den überschüssigen PVP Molekülen aufgebrochen werden. Die Folge dieser Übersättigung ist eine Abnahme der Gesamtzahl an Verknüpfungspunkten und damit eine Abnahme der Gelviskosität. Als weitere Möglichkeit zur Steuerung der Gelviskosität kann die Zugabe von protischen Lösungsmitteln (z.B. Wasser, Alkohole, Amine, Thiole) oder organischen Protonendonatoren (Carbonsäuren z.B. Salicylsäure) oder anorganischen Agentien (z.B. Lewis-Säuren) genutzt werden. Hier bieten sich speziell Verbindungen aus den Substanzklassen der tertiären Polyamine und der Polyamide an. In jedem dieser Fälle trägt die Zugabe der Agentien zur Verringerung der Koordinationsstellen entweder an der Polyacrylsäure oder am PVP bei. Dadurch wird die Zahl der potentiellen Verknüpfungspunkte zur Ausbildung von Gelmaschen verringert, was einen direkten Einfluss auf die Viskosität des Gels hat.

Des weiteren lassen sich die resultierenden Geleigenschaften der Matrices über das Molekulargewicht, Substitutions- und Vernetzungsgrad der eingesetzten Polyacrylsäure beeinflussen.

Zur Ausfertigung besonderer anwendungstechnischer Eigenschaften werden die Gelmatrices mit den entsprechenden Weichmachern, Lösungsvermittlern, Penetrationsenhancem, Füllstoffen und/oder anderen bekannten Zusätzen versetzt.

Als Gelgrundlage wird Polyacrylsäure eingesetzt. Polyacrylate sind vorteilhaft im Sinne der vorliegenden Erfindung zu verwendende gelbildende Polymere. Erfindungsgemäß vorteilhafte Polyacrylate sind Acrylat-Alkylacrylat-Copolymere, insbesondere solche, die aus der Gruppe der sogenannten Carbomere oder Carbopole (Carbopol® ist eigentlich eine eingetragene Marke der B. F. Goodrich Company) gewählt werden. Insbesondere zeichnen sich das oder die erfindungsgemäß vorteilhaften Acrylat-Alkylacrylat-Copolymere durch die folgende Struktur aus:

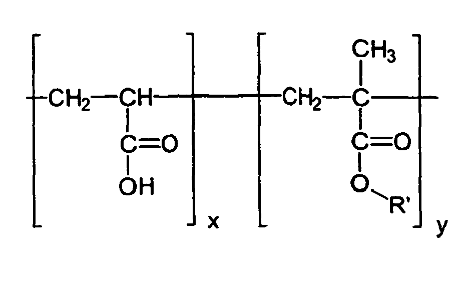

Darin stellen R' einen langkettigen Alkylrest und x und y Zahlen dar, welche den jeweiligen stöchiometrischen Anteil der jeweiligen Comonomere symbolisieren.

Erfindungsgemäß besonders bevorzugt sind Acrylat-Copolymere und/oder Acrylat-Alkylacrylat-Copolymere, welche unter den Handelbezeichnungen Carbopol® 1382, Carbopol® 981 und Carbopol® 5984 von der B. F.Goodrich Company erhältlich sind, bevorzugt Polyacrylate aus der Gruppe der Carbopole der Typen 980, 981, 1382, 2984, 5984 sowie besonders bevorzugt Carbomer 2001.

Ferner vorteilhaft sind Copolymere aus C₁₀₋₃₀-Alkylacrylaten und einem oder mehreren Monomeren der Acrylsäure, der Methacrylsäure oder deren Ester, die kreuzvemetzt sind mit einem Allylether der Saccharose oder einem Allylether des Pentaerythrit.

Polyacrylsäure und/oder deren Copolymere werden bevorzugt in einer Menge von 5 - 55 Gew.%, besonders bevorzugt zwischen 5 - 30 Gew.% eingesetzt. Alle Prozentangaben beziehen sich dabei Gewichtsanteile Gelmatrix sofern nicht Gegenteiliges angegeben ist.

Als Vernetzer wird Polyvinylpyrrolidon (PVP), z.B. Luviskol der Firma BASF, eingesetzt, bevorzugt in einer Menge von 0,25 - 60 Gew.%, besonders bevorzugt zwischen 1 - 30 Gew.%. In gleichem Maße können auch_PVP-Copolymerisate wie beispielsweise Vinylpyrrolidon-Vinylacetat (Povidonacetat; Kollidon VA 64), Terpolymere auf Basis Vinylpyrrolidon und Acrylsäure oder Methacrylsäure bzw. deren Ester (Luviflex VBM 35), Copolymerisate aus Vinylpyrrolidon und Vinylimidazoliummethochlorid (Luviquat-Marken) als sog. PVP- Vemetzungsagenz eingesetzt werden.

Als weitere Gelbestandteile können Polyalkohol oder -alkohole, z.B. 1,2-Propandiol, Glycerin, und/oder Wasser eingesetzt werden, bevorzugt in einer Menge von 5 - 90 Gew.%, besonders bevorzugt zwischen 5 - 45 Gew.%.

Weitere Bestandteile der Gelmatrix können Lösungsvermittler, z.B. Polyethylenglycole (Lutrol E400, E600 der Firma BASF) in einer Menge von 0 - 50 Gew.%, bevorzugt 0 - 30 Gew.%, Neutralisationsmittel, z.B. Tromethamol, Triethanolamin und/oder Dexpanthenol, in einer Menge 0 - 30 Gew.%, bevorzugt 0 - 15 Gew.%, Füllstoff(e), z.B. Kieselsäure, mikronisierte Cellulose und/oder Gelatine, in einer Menge von 0 - 30 Gew.%, bevorzugt 3 - 15 Gew.%, und natürlich Wirkstoff(e), z.B. Menthol, Jojobaöl, Ibuprofen, Benzylnicotinat und/oder Capsaicin, in einer Menge von 0 - 35 Gew.%, bevorzugt 0 -15 Gew.%, sein.

Die Herstellung dieser Gelmatrices erfolgt bevorzugt lösemittelfrei, vorzugsweise bei Raumtemperatur, in handelsüblichen Knetern oder geeigneten Extrudern.

Für eine monolithische Pflasteranwendung vereint die erfindungsgemäße Gelmatrix auf Polyacrylsäurebasis die nötige Klebkraft mit der entsprechenden Kohäsivität. Zur Anwendung als Pflaster werden die Gelmatrices als Schicht auf ein Trennmedium aus Papier, Folie o. ä. gepresst, gewalzt o. ä. und auf der Rückseite mit einem beliebigen Trägermaterial kaschiert. Besonders vorteilhaft ist die erfindungsgemäße Gelmatrix auf einer flexiblen Deckschicht aufgebracht, insbesondere bei der Verwendung als Pflaster. Aufgebaut ist ein entsprechendes Pflaster aus einem Träger wie Folien, Vliese, Gewebe, Schäume etc., der Klebmatrix und Abdeckfolie, Abdeckpapier oder Trennpapier zum Schutz der klebenden Matrix vor dem Gebrauch des Pflasters.

In einer weiteren bevorzugten Ausführungsform der Erfindung werden als Träger Polymerfolien, Vliese, Gewebe sowie deren Kombinationen eingesetzt. Als Trägermaterialien stehen u.a. Polymere wie Polyethylen, Polypropylen und Polyurethan oder auch Naturfasern zur Auswahl.

Zusammenfassend kann festgehalten werden, dass als Trägermaterialien sich alle starren und elastischen Flächengebilde aus synthetischen und natürlichen Rohstoffen eignen. Bevorzugt sind Trägermaterialien, die so eingesetzt werden können, dass sie Eigenschaften eines funktionsgerechten Verbandes erfühlen. Beispielhaft sind Textilien wie Gewebe, Gewirke, Gelege, Vliese, Laminate, Netze, Folien, Schäume und Papiere aufgeführt. Weiter können diese Materialien vor- beziehungsweise nachbehandelt werden. Gängige Vorbehandlungen sind Corona und Hydrophobien; geläufige Nachbehandlungen sind Kalandern, Tempern, Kaschieren, Stanzen und Eindecken.

Besonders vorteilhaft ist, wenn das Trägermaterial sterilisierbar, bevorzugt γ-(gamma) sterilisierbar, ist.

Die genannten Eigenschaften der Klebmatrix legen insbesondere die Verwendung für medizinische Produkte, insbesondere Pflaster, medizinische Fixierungen, Wundabdeckungen, orthopädische oder phlebologische Bandagen und Binden nahe.

Schließlich kann die Gelmatrix mit einem klebstoffabweisenden Trägermaterial, wie silikonisiertes Papier, eingedeckt oder mit einer Wundauflage oder einer Polsterung versehen werden. Auf seiner selbstklebend ausgerüsteten, später der Haut zugewandten Seite ist das erfindungsgemäße Pflaster über seine ganze Breite bis zum Gebrauch üblicherweise mit einem klebstoffabweisenden Trägermaterial abgedeckt. Dieses schützt die Selbstklebeschicht aus der gut hautverträglichen Klebemasse der Gelmatrix, die vorzugsweise im Transferverfahren aufgebracht worden ist, und stabilisiert zusätzlich das ganze Produkt. Die Abdeckung kann in bekannter Weise einstückig oder vorzugsweise zweiteilig ausgebildet sein.

Weitere Ausführungsformen können dergestalt sein, daß zwischen der Rückseite der Matrix und dem Abdeckträger sich eine zweite Matrix mit höherer Wirkstofflöslichkeit als Reservoir befindet. Dies könnte statt einer zweiten Matrix und Träger auch eine Tiefziehfolie mit reinem Wirkstoff sein.

Auf der Klebseite der Matrix befindet sich teilweise (z.B. am Rand) eine zweite Matrix mit hoher Klebkraft zur zusätzlichen Fixierung, aber ungenügender Wirkstofflöslichkeit.

Die wirkstofffreie Matrix befindet sich zwischen zwei nicht verankernden Folien und wird zur Fixierung von Elektroden etc., oder wegen der Wasseraufnahmefähigkeit bei entsprechender Geometrie von Colostomie-/Ileostomiebeuteln genutzt. Die wirkstofffreie Matrix könnte auch (mit oder ohne Wundauflage) als Klebschicht für ein einfaches Wund-/Heftpflaster dienen.

Die nachfolgenden Beispiele veranschaulichen die Erfindung ohne sie einzuschränken. In der nachfolgenden Tabelle sind die erfindungsgemäßen Gelmatrices aufgeführt.

### Beispiele 1 bis 9

| Bestandteile/Beispiel | 1 | 2 | 3 | 4 | 5 | 6 | 7 | 8 | 9 |
|---|---|---|---|---|---|---|---|---|---|
| Polyacrylsäure | 22,5 % | 18,0 % | 22,5 % | 22,5 % | 22,5 % | 22,5 % | 22,5 % | 21,0 % | 21,0 % |
| Polyvinylpyrrolidon, PVP 25 | 3,5% | 3,5% | 3,5% | 3,5% | 3,5% | 3, 5% | 3,5% | 3,5% | 3,5% |
| Propandiol | 36,0 % | 43,5 % | 36,5 % | 37,5 % | 35,5 % | 25,5 % | 40,0 % | 35,5 % | 35,5 % |
| Polyethylenglycol | 17,0 % | 23,5 % | 17,0 % | 6,5% | 20,0 % | 12,0 % | 21,0 % | 20,0 % | 20,0 % |
| Kieselsäure | 8,0% | 5,0% | 10,0 % | 10.0 % | 11,5 % | 11.5 % | 10,0 % | 10,0 % | 10,0 % |
| Dexpanthenol | 5,0% | 5,0% | 5.0% | 5,0% | 5,0% | 5,0% | - | 5,0% | 5.0% |
| Jojobaöl | 5,0% | 0,5% | 5,0% | 5,0% | - | - | - | - | - |
| Salicylsäure | 3,0% | 1,0% | - | - | - | - | 3,0% | - | - |
| Benzylnicotinat | - | - | 0,5% | - | - | - | - | - | - |
| Glycolsalicylat | - | - | - | 10,0 % | - | 10,0 % | - | - | - |
| Menthol | - | - | - | - | 2,0% | - | - | - | - |
| Pfefferminzöl | - | - | - | - | - | 10,0 % | - | - | - |
| Ibuprofen | - | - | - | - | - | - | - | 5,0% | - |
| Teebaumöl | - | - | - | - | - | - | - | - | 5,0% |

### Beispiele 10 bis 18

| Bestandteile/Beispiele | 10 | 11 | 12 | 13 | 14 | 15 | 16 | 17 | 18 |
|---|---|---|---|---|---|---|---|---|---|
| Polyacrylsäure | 5,0% | 10,5 % | 10,5 % | 21,0 % | 21,0 zip | 21,0 % | 10.5 % | 11,7 % | 18,0 % |
| Polyvinylpyrrolidon | 30,0 % | 3,5% | 3,5% | 3,5% | 3,5% | 3,5% | 3,5% | 3,9% | 3,5% |
| Propandiol | - | - | 5,0% | 35,5 % | 35,5 % | 35,5 % | - | - | 58,5 % |
| Polyethylenglycol | - | - | - | 15,0 % | 20,0 % | 20,0 % | 20,0 % | 22,1 % | - |
| Diethylenglycol | 25,0 % | - | - | - | - | - | - | - | - |
| Glycerin | - | 72,47 % | 77,0 % | - | - | - | 46,0 % | 51,1 % | 10,0 % |
| Wasser | 15,0 % | - | - | - | - | - | - | - | - |
| Polyoxyethylen(20)-sorbitanmonolaurat | - | 10, 0 % | - | - | - | - | - | - | - |
| Isopropylmyristat | - | - | - | 5,0% | - | - | - | - | - |
| Kieselsäure | 15,0 % | - | - | 10,0 % | 10,0 % | 10,0 % | 10,0 % | - | - |
| Gelatine | - | 3,5% | 3,5% | - | - | - | - | - | - |
| Dexpanthenol | - | - | - | 5,0% | 5,0% | 5,0% | 5,0% | 5,6% | 5,0% |
| Harnstoff | 10,0 % | - | - | - | - | - | - | - | - |
| Vitamin A-palmitat | - | 0,03 % | - | - | - | - | - | - | - |
| Capsicum-Extrakt | - | - | 0,5% | 5,0% | - | - | - | - | - |
| Clotrimazol | - | - | - | - | 5,0% | - | - | - | - |
| Lidocain HCl | - | - | - | - | - | 5,0% | - | - | - |
| Ibuprofen | - | - | - | - | - | - | 5,0% | 5,6% | 5,0% |

Zur Anwendung als Pflaster sind alle beispielhaft ausgeführten Gelmatrices als Schicht auf ein Trennmedium (Träger) aus Papier und Folie gewalzt worden und ihre Klebeigenschaft und Kohäsivität sensorisch begutachtet worden. Bei allen erfindungsgemäßen Pflastern ergaben sich zum Unterschied zu Pflastern mit bekannten Gelen eine ausreichend gute Klebkraft und eine entsprechende Kohäsivität, so dass sich alle Pflaster rückstandslos von der Haut abziehen ließen.

### Beispiele 19 bis 25

Entsprechend Beispiel 1 wurden anstelle des Polyvinylpyrolidon PVP 25 folgende Polyvinylpyrrolidone bzw. Mischungen daraus eingesetzt.

| Beispiel | | Mittleres Molekulargewicht [g/mol] |
|---|---|---|
| 19 | PVP 12 | ca. 2 500 |
| 20 | PVP 17 | ca. 11 500 |
| 21 | PVP 25 | ca. 25 000 |
| 22 | PVP 30 | ca. 40 000 |
| 23 | PVP 90 | ca. 700 000 |
| 24 | PVP 12 und PVP 30, 1:1 | ca. 21 250 |
| 25 | PVP 25 und PVP 90, 1:1 | ca. 370 000 |

Beispiel 19, 20, 21 und 24 führte zu Gelen, deren Klebkraft gut bzw. sehr gut und derenKohäsivität als ausreichend beurteilt werden konnte. Beispiel 22, 23 und 25 führte zu Gelen, die gering viskos waren und eine geringere Klebkraft aufwiesen. Der Vorteil bei der erfindungsgemäßen Art der Vernetzung liegt somit darin, über die ParameterAnteil-PVP und Molekulargewicht-PVP zielgerichtet Gelmatrices herzustellen, deren Klebrigkeit, Kohäsivität und Viskosität individuell auf den jeweiligen Anwendungsbereich einstellbar sind
Die vorteilhaften Eigenschaften der erfindungsgemäßen Gelmatrices in transdermalen Systemen sind damit eindrucksvoll bestätigt worden.

## Patentansprüche

1. Selbstklebende Gelmatrix auf Polyacrylsäurebasis erhältlich durch die Vernetzung der Polyacrylsäure mit Polyvinylpyrrolidon (PVP) und/oder deren Copolymeren über die Bindung quartärer Ammoniumsalze des Polyvinylpyrrolidon (PVP) mit der Hydroxyfunktion der Polyacrylsäure.

2. Gelmatrix nach Anspruch 1, **dadurch gekennzeichnet, dass** PVP und/oder deren Copolymere und/oder Mischungen daraus mit verschiedenen mittleren Molekulargewichten als Vernetzungsmittel der Polyacrylsäure eingesetzt werden.

3. Gelmatrix nach Anspruch 1 oder 2, **dadurch gekennzeichnet, dass** das oder die mittleren PVP-Molekulargewichte im Bereich zwischen 2500 und 700000 g/mol liegen.

4. Gelmatrix nach Anspruch 1, 2 oder 3, **dadurch gekennzeichnet, dass** das Gel 5 - 55 Gew.%, bevorzugt 5 - 30 Gew.%, Polyacrylsäure und/oder deren Copolymere und 0,25 - 60 Gew.%, bevorzugt 1 - 30 Gew.% PVP und/oder deren Copolymere umfasst.

5. Gelmatrix nach einem der vorherigen Ansprüche, **dadurch gekennzeichnet, dass** die Gelmatrix Polyalkohol, insbesondere Propandiol, Polyethylenglycol und/oder Glycerin, in einer Menge von 5 - 90 Gew.%, besonders bevorzugt zwischen 5 - 45 Gew.%, umfasst.

6. Gelmatrix nach einem der vorherigen Ansprüche, **dadurch gekennzeichnet, dass** das Gel protische Lösungsmittel, insbesondere Wasser, Alkohole, Amine oder Thiole, und/oder organische Protonendonatoren, insbesondere Salicylsäure, und/oder anorganische Agentien, wie z.B. Lewis-Säuren, umfasst.

7. Gelmatrix nach einem der vorherigen Ansprüche, **dadurch gekennzeichnet, dass** das Gel mit hydrophoben Wirkstoffen und einem Lösungsvermittler oder/und mit hydrophilen Wirkstoffen dotiert ist.

8. Gelmatrix nach Anspruch 7, **dadurch gekennzeichnet, dass** das Gel mit Dexpanthenol, Jojobaöl, Salicylsäure, Benzylnicotinat, Glycolsalicylat, Menthol, Pfefferminzöl, Ibuprofen, Teebaumöl, Harnstoff, Vitamin A-palmitat, Capsicum-Extrakt, Clotrimazol, und/oder Lidocain HCl dotiert ist.

9. Gelmatrix nach einem der vorherigen Ansprüche, **dadurch gekennzeichnet, dass** das Gel mit Weichmachern, Lösungsvermittlern, Penetrationsenhancem, Füllstoffen und/oder anderen geeigneten Zusätzen versetzt wird.

10. Verwendung der Gelmatrix nach Anspruch 1 bis 9 in medizinischen Fixierungen, Wundabdeckungen, orthopädische oder phlebologische Bandagen und/oder Binden.

## Claims

1. Self-adhesive polyacrylic acid-based gel matrix obtainable by crosslinking the polyacrylic acid with polyvinylpyrrolidone (PVP) and/or copolymers thereof via bonding of quaternary ammonium salts of polyvinylpyrrolidone (PVP) with the hydroxyl function of the polyacrylic acid.

2. Gel matrix according to Claim 1, **characterized in that** PVP and/or copolymers thereof and/or mixtures thereof with various average molecular weights are employed as crosslinkers of the polyacrylic acid.

3. Gel matrix according to Claim 1 or 2, **characterized in that** the average PVP molecular weight(s) are in the range between 2500 and 700 000 g/mol.

4. Gel matrix according to Claim 1, 2 or 3, **characterized in that** the gel comprises 5-55% by weight, preferably 5-30% by weight, of polyacrylic acid and/or copolymers thereof and 0.25-60% by weight, preferably 1-30% by weight, of PVP and/or copolymers thereof.

5. Gel matrix according to any of the preceding claims, **characterized in that** the gel matrix comprises polyalcohol, in particular propanediol, polyethylene glycol and/or glycerol, in an amount of 5-90% by weight, particularly preferably between 5-45% by weight.

6. Gel matrix according to any of the preceding claims, **characterized in that** the gel comprises protic solvents, in particular water, alcohols, amines or thiols, and/or organic proton donors, in particular salicylic acid, and/or inorganic agents such as, for example, Lewis acids.

7. Gel matrix according to any of the preceding claims, **characterized in that** the gel is doped with hydrophobic active ingredients and a solubilizer or/and with hydrophilic active ingredients.

8. Gel matrix according to Claim 7, **characterized in that** the gel is doped with dexpanthenol, jojoba oil, salicylic acid, benzyl nicotinate, glycol salicylate, menthol, peppermint oil, ibuprofen, tea tree oil, urea, vitamin A palmitate, capsicum extract, clotrimazole, and/or lidocaine HCl.

9. Gel matrix according to any of the preceding claims, **characterized in that** the gel is mixed with plasticizers, solubilizers, penetration enhancers, fillers and/or other suitable additives.

10. Use of the gel matrix according to Claims 1 to 9 in medical fixings, wound coverings, orthopedical or phlebological bandages and/or dressings.

## Revendications

1. Matrice de gel autoadhésive à base de poly(acide acrylique), pouvant être obtenue par la réticulation du poly(acide acrylique) avec de la polyvinylpyrrolidone (PVP) et/ou des copolymères de celle-ci, par la liaison de sels d'ammonium quaternaire de la polyvinylpyrrolidone (PVP) avec à fonction hydroxy du poly(acide acrylique).

2. Matrice de gel selon la revendication 1, **caractérisée en ce qu'**en tant qu'agent de réticulation du poly(acide acrylique) on utilise de la PVP et/ou des copolymères de celle-ci et/ou des mélanges de ceux-ci ayant différentes masses moléculaires moyennes.

3. Matrice de gel selon la revendication 1 ou 2, **caractérisée en ce que** la ou les masse(s) moléculaire(s) moyenne(s) de la PVP se situe(nt) dans la plage comprise entre 2 500 et 700 000 g/mole.

4. Matrice de gel selon la revendication 1, 2 ou 3, **caractérisée en ce que** le gel comprend 5 - 55 % en poids, de préférence 5 - 30 % en poids, de poly(acide acrylique) et/ou de copolymères de celui-ci et 0,25 - 60 % en poids, de préférence 1 - 30 % en poids de PVP et/ou de copolymères de celle-ci.

5. Matrice de gel selon l'une quelconque des revendications précédentes, **caractérisée en ce que** la matrice de gel comprend un polyalcool, en particulier du propanediol, du polyéthylèneglycol et/ou du glycérol, en une quantité de 5 - 90 % en poids, de façon particulièrement préférée comprise entre 5 et 45 % en poids.

6. Matrice de gel selon l'une quelconque des revendications précédentes, **caractérisée en ce que** le gel comprend des solvants protiques, en particulier de l'eau, des alcools, des amines ou des thiols, et/ou des donneurs organiques de protons, en particulier de l'acide salicylique, et/ou des agents inorganiques, comme par exemple des acides de Lewis.

7. Matrice de gel selon l'une quelconque des revendications précédentes, **caractérisée en ce que** le gel est doté de substances actives hydrophobes et d'un tiers-solvant et/ou de substances actives hydrophiles.

8. Matrice de gel selon la revendication 7, **caractérisée en ce que** le gel est doté de dexpanthénol, d'huile de jojoba, d'acide salicylique, de nicotinate de benzyle, de salicylate de glycol, de menthol, d'essence de menthe poivrée, d'ibuprofène, d'essence de théier, d'urée, de palmitate de vitamine A, d'extrait de *Capsicum,* de clotrimazole et/ou de lidocaïne HCl.

9. Matrice de gel selon l'une quelconque des revendications précédentes, **caractérisée en ce que** le gel est additionné de plastifiants, de tiers-solvants, d'agents de pénétration, de charges et/ou d'autres additifs appropriés.

10. Utilisation de la matrice de gel selon l'une quelconque des revendications 1 à 9 dans des fixations médicales, des pansements, des bandes et/ou bandages orthopédiques ou phlébologiques.
